# EUROPÄISCHE PATENTANMELDUNG

(11) **EP 0 557 708 A1**
(43) Veröffentlichungstag der Anmeldung: **01.09.1993**
(21) Anmeldenummer: 93100661.3
(22) Anmeldetag: 18.01.1993
(51) Int. Cl.: A61K 31/00, A61K 31/385, A61K 31/195

(54) **Arzneimittel die Liponsäure und Pantothensäure enthalten**

(30) Priorität: 18.01.1992 DE 4201196
(71) Anmelder: Rabien, Margarethe, 65239 Hochheim (DE)
(72) Erfinder:

(57) **Zusammenfassung**

Kombinationspräparat mit den Hauptbestandteilen α-Liponsäure + Pantothensäure (bzw. Dexpanthenol) als Arzeneimittel gegen Ataxie, Multiple Sklerose, Polyneuropathie, Muskelatrophie, Muskeldystrophie und ähnliche Krankheiten.

## Beschreibung

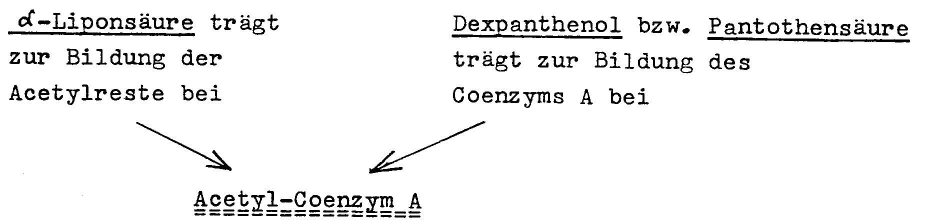

Ausgangssubstanz des Zitronensäurezyklus = wichtigste Energiequelle aller Zellen und des peripheren Nervensystems.

Ideale Behandlungsmethode gegen Multiple Sklerose, Ataxie, Muskelatrophie, Muskelhypotonie und evtl. auch Muskeldystrophie.

Die Kombination beider Präparate regt den Zellstoffwechsel an und gleicht neurologische Symptome aus. Besonders durch die Bildung von mehr ATP (chemischer Energieträger) und mehr Acetylcholin (Überträgersubstanz von Nerven auf den Muskel).

Mehrkomponentenmedikament (bzw. Mehrkomponentenpackumg) gegen Polyneuropathie Ataxie, Muskelatrophie usw., dadurch gekennzeichnet, daß es folgende Kombinationen enthält:
¹⁾ α-Liponsäure = Coenzym der oxidativen Decarboxilierung.

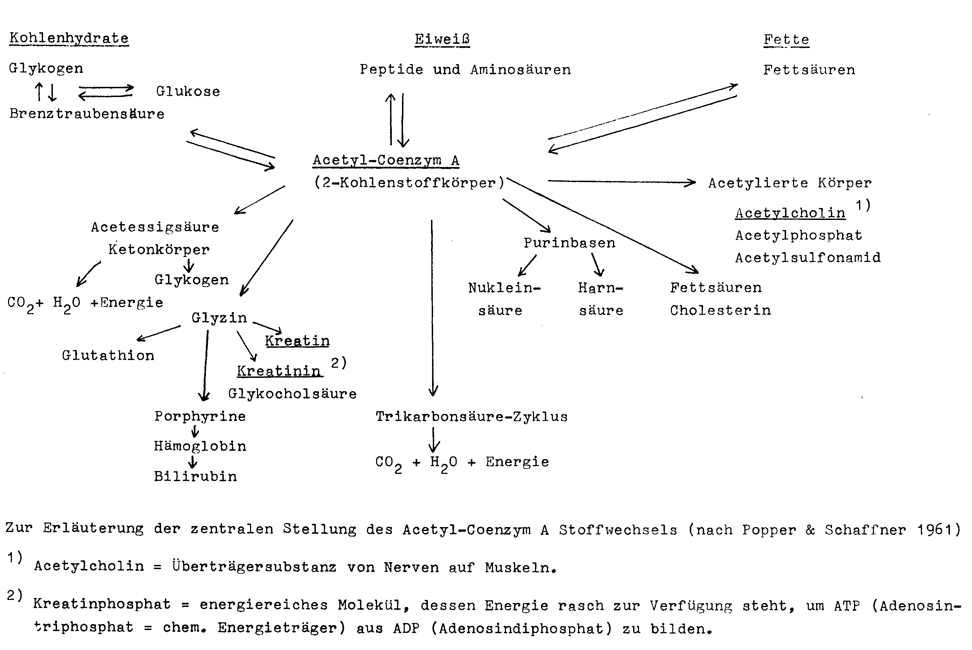

## Patentansprüche

1. Pantothensäure (bzw. Dexpanthenol), α-Liponsäure¹⁾

2. Vitamin B2-Komplex, α-Liponsäure

3. Pantothensäure (bzw. Dexpanthenol), α-Liponsäure, Vitamin B1, B6, B12

4. Pantothensäure (bzw. Dexpanthenol), α-Liponsäure, Pyridoxin (B6)

5. B2-Komplex, α-Liponsäure, Vitamin B1, B6, B12.

6. Pantothensäure (bzw. Dexpanthenol), α-Liponsäure + Vitamin E

7. B2-Komplex, α-Liponsäure, Vitamin B1, B6, B12 + Vitamin E
